# EUROPEAN PATENT SPECIFICATION

(11) **EP 0 855 178 B1**
(45) Date of publication and mention of the grant of the patent: **09.06.2004**
(21) Application number: 98101068.9
(22) Date of filing: 22.01.1998
(51) Int. Cl.: A61K 7/06

(54) **Hair care products comprising an alpha-hydroxy acid and a silicone elastomer powder**
Haarpflegemittel, die eine alpha-Hydroxysäure und ein Silikon-Elastomer Pulver enthalten
Produits de soins capillaires contenant un alpha-hydroxyacide et une poudre de silicone élastomérique

(30) Priority: 27.01.1997 JP 1235797
(43) Date of publication of application: 29.07.1998
(73) Proprietor: KAO CORPORATION, Chuo-ku, Tokyo (JP)
(72) Inventor: Tanihara, Mamoru, c/o Kao Corporation, Sumida-ku, Tokyo (JP); Kasuga, Fumiko, c/o Kao Corporation, Sumida-ku, Tokyo (JP)
(74) Representative: Kindler, Matthias, Dr. Dipl.-Chem.

(56) References cited:
- EP-A- 0 240 349
- EP-A- 0 240 350
- EP-A- 0 435 483
- PATENT ABSTRACTS OF JAPAN vol. 095, no. 001, 28 February 1995 & JP 06 298625 A (KAO CORP), 25 October 1994

## Description

This invention relates to hair care products. More particularly, it relates to hair care products which can be washed away after using which can impart softness, smoothness and good combing properties to the moist hair and, after drying, make the hair soft and well styled while keeping smooth feel and exerting long-lasting conditioning effects thereon.

There have been required various hair care products having conditioning effects. In particular, it has been required to develop hair care products capable of imparting softness and smoothness to the moist hair and, after drying, smooth and soft feel and also long-lasting effects thereof to the hair. For example, JP-A-8-34710 proposes a hair care product which contains specific surfactants (anionic/imidazoline type ampholytic surfactants) and an α-hydroxycarboxylic acid as a conditioning agent (the term "JP-A" as used herein means an "unexamined published Japanese patent application"). However, this product is still unsatisfactory in the effects on the moist hair and smooth feel and long-lasting effects after drying the hair.

JP-A-6-80559 discloses a detergent composition which comprises a water-insoluble polymer powder (e.g., a silicone resin) and a water-insoluble, nonvolatile silicone (liquid or paste) dispersed in an aqueous solution of a surfactant. However, this composition fails to impart sufficient softness and moistening effects to the hair.

EP 0 240 350 A2 describes hair care compositions leading to improved hair conditioning and style retention properties. These compositions comprise particular types of silicon polymers and volatile carriers for such polymers. These polymers are rigid silicone polymers.

JP 06298625 is directed to silicone derivatives that are used in a hair treatment composition to improve the setting property and the comb-ability. The composition comprises also a hydroxy-acid and the silicones include e.g. dimethylpolysiloxane.

### SUMMARY OF THE INVENTION

Accordingly, an object of the present invention is to provide hair care products which can impart softness and smoothness to the moist hair and, after drying, exert long-lasting conditioning effects so as to make the hair smooth, soft, moist and well styled.

Under these circumstances, the present inventors have conducted extensive studies. As a result, they have found that the combined use of α-hydroxycarboxylic acids with specific water-insoluble silicone elastomer powders as defined in claim 1 makes it possible to provide hair care products to be washed away after using which can impart softness and smoothness to the moist hair and, after drying, make the hair soft, moist and well styled while keeping smooth feel and exerting long-lasting conditioning effects thereon, thus completing the present invention.

Accordingly, the present invention provides hair care products which comprise α-hydroxycarboxylic acids and water-insoluble silicone elastomer powders as defined in claim 1 and which can be washed away after using.

The α-hydroxycarboxylic acids to be used in the present invention comprise mono- or polycarboxylic acids having one or more hydroxyl functional groups at least one of which is introduced into the α-position (i.e., on the carbon atom adjacent to the carboxyl functional group).

Examples of these compounds include citric acid, lactic acid, methyllactic acid, phenyllactic acid, malic acid, mandelic acid, glycolic acid, tartronic acid, tartaric acid and gluconic acid. Among these compounds, it is preferable to use citric acid, lactic acid, malic acid, glycolic acid or tartaric acid therefor.

In the present invention, use may be made of either one of these α-hydroxycarboxylic acids or a combination of two or more of the same. The content of the α-hydroxycarboxylic acid(s) preferably ranges from 0.01 to 10 % by weight, more preferably from 0.5 to 10 % by weight, based on the whole composition, since the softness of the hair is improved thereby.

The water-insoluble silicone elastomer rubber powders are selected from methylpolysiloxanes, which are obtainable by polymerizing or copolymerizing methylhydrogenpolysiloxane, methylvinylpolysiloxane and/or α,ω-divinyldimethylpolysiloxane and have rubber elasticity and crosslinked structure, and derivatives thereof. The methylpolysiloxane derivatives are selected from amino-modified methylpolysiloxane, phenyl-modified methylpolysiloxane and epoxy-modified methylpolysiloxane. These water-insoluble silicone elastomer rubber powders are called silicone rubber powders which are different from silicone resin powders having a true specific gravity of about 1.3.

The water-insoluble silicone elastomer powder may be a composite powder comprising the above-described methylpolysiloxane or derivative thereof as the major component together with other water-insoluble polymer powder(s). Moreover, use may be made therefor of methylpolysiloxane or derivative thereof coated with a water-insoluble polymer by a conventional method. (Hereinafter, they will be sometimes referred to as "composite silicone elastomer powders".) The water-insoluble polymer as used herein includes polymethylsilsesquioxanes; amorphous silicon dioxide; polyethylene, polypropylene, polystyrene, polyamide, polyester or styrene/divinylbenzene copolymers.

The water-insoluble silicone elastomer powder has an average particle size of preferably from 0.01 to 100 µm, more preferably from 0.1 to 30 µm. The particles thereof may have a spherical, oval or spindle shape. Among these, spherical shape is preferable and particularly has a major axis/short axis ratio of 2 or below, preferably 1.3 or below.

The water-insoluble silicone elastomer powder has a true specific gravity of from. 0.9 to 1.2, preferably from 0.9 to 1.1. Further, it is preferable that the water-insoluble silicone elastomer powder has a rubber hardness (determined according to JIS K6253 using Type A Durometer) of from 5 to 95, more preferably from 10 to 90.

Although the water-insoluble silicone elastomer powder may be used as such, it may be employed in the form of a surfactant-containing emulsion having the powder dispersed therein so as to improve the stability and handling properties.

Examples of the water-insoluble silicone elastomer powder include commercially available products such as KMP-598, KMP-597 and X-52-1139G (each manufactured by Shin-Etsu Chemical Co., Ltd.) and Trefil E-500, E-501, E-600, E-602, E-850 and E-730S (each manufactured by Dow Corning Toray Silicone Co., Ltd.).

Use may be made of either one of these water-insoluble silicone elastomer powders or a combination of two or more of the same.

The content of the water-insoluble silicone elastomer powder(s) preferably ranges from 0.01 to 10 % by weight, more preferably from 0.05 to 5 % by weight, based on the whole composition, since the smoothness and combing properties of the moist hair and the smooth feel and the persistence of the conditioning effects after drying the hair is improved - thereby.

The hair care product of the present invention may further contain surfactant(s). These surfactants are not particularly restricted but arbitrarily selected from those commonly employed in cosmetics.

To produce shampoo compositions, use can be made of one or more surfactants selected from anionic, ampholytic and nonionic surfactants as the major component optionally together with cationic surfactant. The content of the surfactant(s) in the composition preferably ranges from 5 to 30 % by weight, in particular, 10 to 25 % by weight, based on the whole composition.

To produce rinses or hair conditioners, use can be made of one or more surfactants selected from cationic and nonionic surfactants as the major component optionally together with ampholytic and anionic surfactants. The content of the surfactant(s) in the composition preferably ranges from 0.1 to 20 % by weight, in particular, 0.5 to 10 % by weight, based on the whole composition.

In addition to the components described above, the hair care products of the present invention may optionally contain components commonly employed in cosmetics, such as oily components such as hydrocarbon oils, vegetable oils, fatty acids, ester oils, perfluoropolyethers and silicone derivatives; medicinal components such as antidandruff agents, bactericides and vitamins; preservatives such as paraben; thickeners such as water-soluble polymers; coloring matters such as dyes and pigments; conditioning agents such as cationic polymers; pearling agents such as glycol esters; and those listed in ENCYCLOPEDIA OF CONDITIONING RINSE INGREDIENTS (MICELLE PRESS, 1987), so long as the effects of the present invention are not deteriorated thereby.

The hair care products of the present invention can be produced by blending the components in a conventional manner. The hair care products of the present invention may be in the form of products to be washed away after using, for example, shampoo compositions such as shampoos and rinse-in-shampoos, rinses, hair treatments, hair conditioners and hair packs.

### EFFECTS OF THE INVENTION

The hair care products of the present invention can impart softness, smoothness and good combing properties to the moist hair and, after drying, make the hair soft, moist and well styled while keeping smooth or oil-free feel and exerting long-lasting conditioning effects thereon.

### [Examples]

To further illustrate the present invention in greater detail, and not by way of limitation, the following Examples will be given.

### Example 1

A shampoo composition as shown in Table 1 was produced in the conventional manner. After using it, evaluation was made on the softness and slipperiness of the moist hair, the softness, moist feel, smooth feel and slipperiness of the hair after drying and the persistence of the conditioning effects. The results are shown in Table 1.

### (Evaluation method)

### (1) Textures of moist hair and dry hair

2 g of the shampoo was applied onto a human tress (weight: about 25 g, length: about 20 cm). After foaming the shampoo for 1 minutes, the tress was rinsed with running water for 30 seconds and then towel-dried. Then the texture of the moist hair and that of the hair dried with a dryer were organoleptically evaluated by skilled panelists in accordance with the following criteria.
E: excellent.
G: good.
M: moderate.
B: bad.

### (2) Persistence of conditioning effect

The hair was shampooed, rinsed and towel-dried in the conventional manner and then the persistence of the conditioning effects (expressed in the change in the texture of the hair after 24 hours) was organoleptically evaluated by skilled panelists in accordance with the following criteria.
E: excellent; long-lasting effects.
G: good; lasting effects.
M: moderate.
B: bad; non-lasting effects.

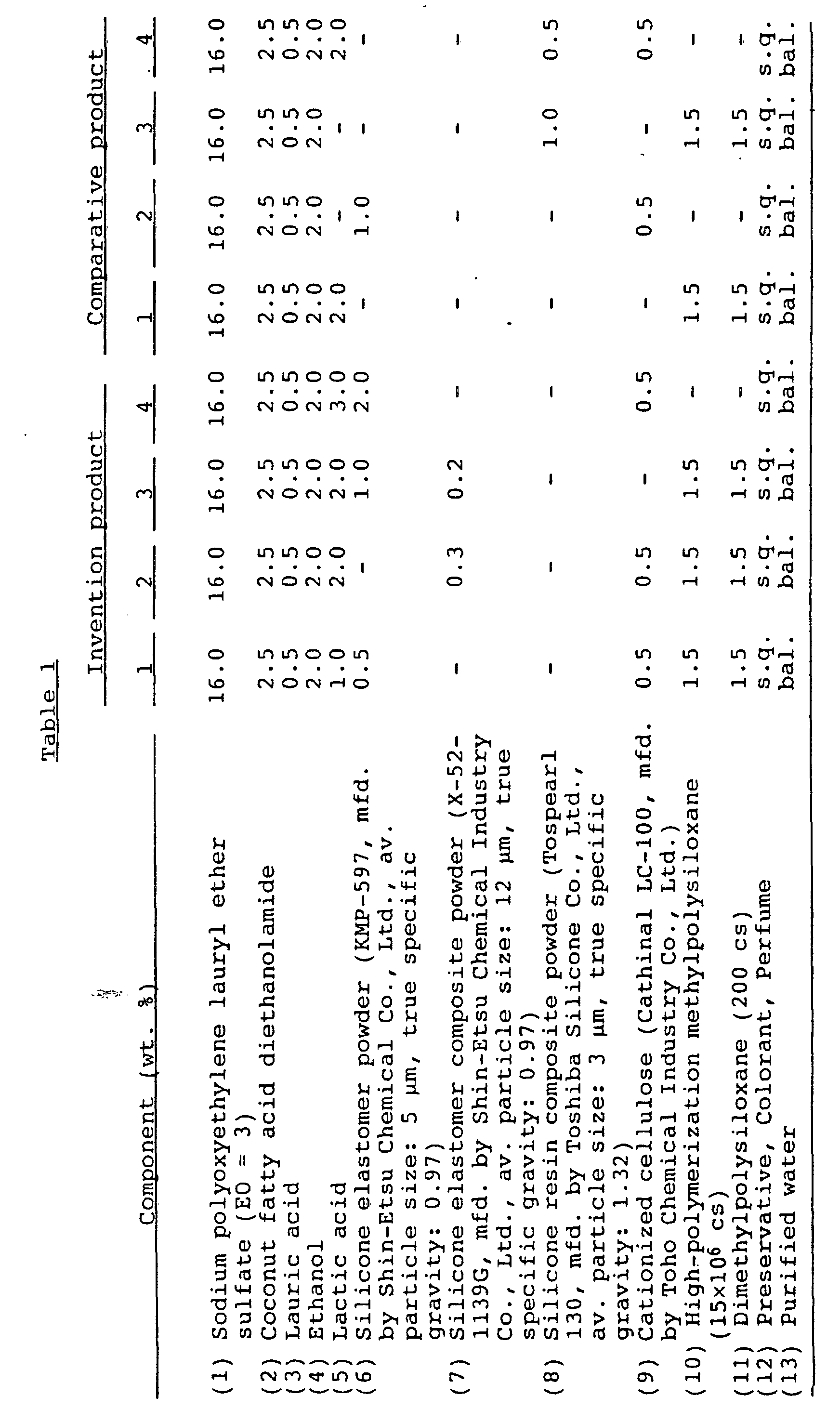

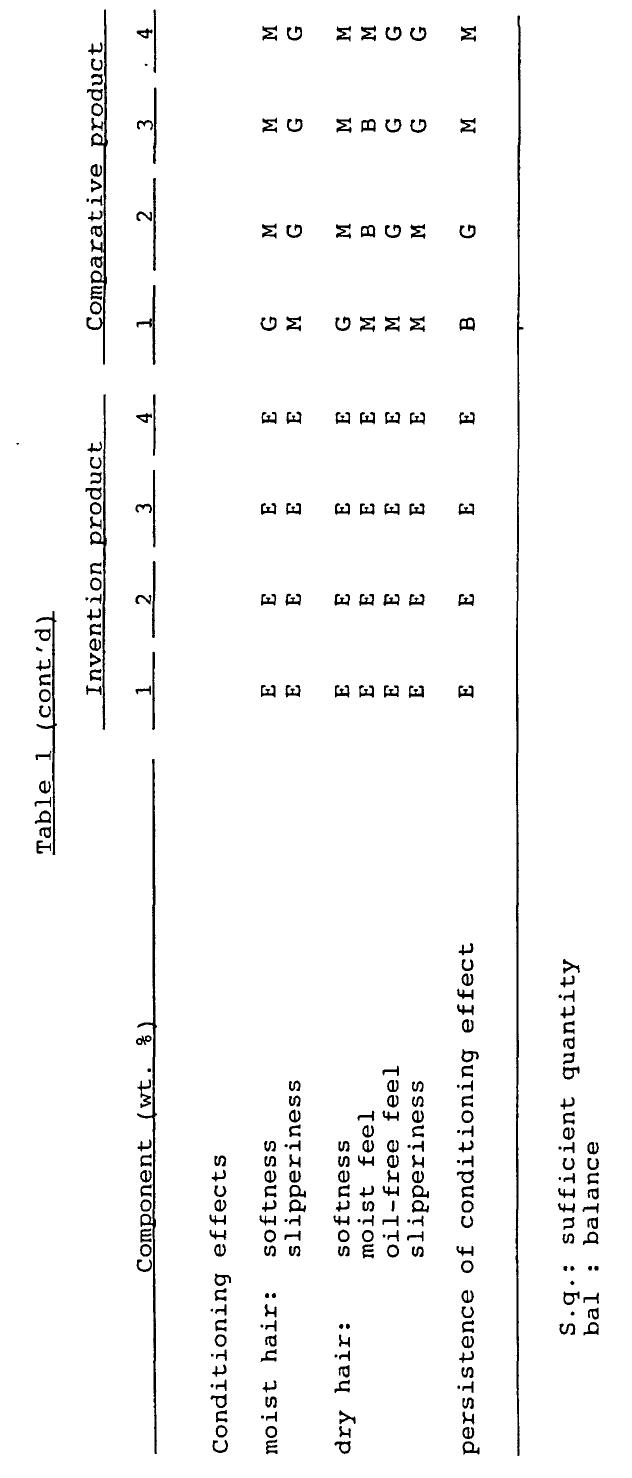

### Example 2 (Shampoo)

A shampoo having the following composition was produced in the conventional manner.

| (Component) | | (wt%) |
|---|---|---|
| (1) | Sodium polyoxyethylene lauryl ether sulfate (EO = 3) | 15.0 |
| (2) | Coconut fatty acid diethanolamide | 3.0 |
| (3) | Amidopropylbetaine laurate | 2.0 |
| (4) | Ethylene glycol distearate | 3.0 |
| (5) | Glycolic acid | 2.5 |
| (6) | Silicone elastomer powder (Trefil E-500, mfd. by Dow Corning Toray Silicone Co., Ltd., average particle size: 3 µm, true specific gravity: 0.97) | 1.0 |
| (7) | Cationic polymer (Merquat 550, mfd. by Calgon Corporation) | 0.3 |
| (8) | High polymerization methylpolysiloxane (6×10⁶ cs) | 2.0 |
| (9) | Dimethylpolysiloxane (200 cs) | 1.5 |
| (10) | Ethanol | 2.0 |
| (11) | Preservative, Colorant, Perfume | sufficient quantity |
| (12) | Purified water | balance |

### Example 3 (Shampoo)

A shampoo having the following composition was produced in the conventional manner.

| (Component) | | (wt%) |
|---|---|---|
| (1) | Ammonium lauryl sulfate | 17.0 |
| (2) | Coconut fatty acid diethanolamide | 2.0 |
| (3) | Amidoamino acid triethanolamine | 2.0 |
| (4) | Ethylene glycol distearate | 3.0 |
| (5) | Lactic acid | 1.5 |
| (6) | Citric acid | 1.0 |
| (7) | Silicone elastomer powder (Trefil E-501, mfd. by Dow Corning Toray Silicone Co., Ltd., average particle size: 10 µm, true specific gravity: 0.97) | 1.0 |
| (8) | Cationic polymer (Polymer JR-400, mfd. by Union Carbide Co., Ltd.) | 0.2 |
| (9) | Stearyltrimethylammonium chloride | 0.1 |
| (10) | High polymerization methylpolysiloxane (1×10 ⁷ cs) | 1.8 |
| (11) | Dimethylpolysiloxane (200 cs) | 1.5 |
| (12) | Ethanol | 2.0 |
| (13) | Preservative, Colorant, Perfume | sufficient quantity |
| (14) | Purified water | balance |

### Example 4 (Hair conditioner)

A hair conditioner of the following composition was produced in the conventional manner.

| (Component) | | (wt%) |
|---|---|---|
| (1) | Cetostearyltrimethylammonium chloride | 2.0 |
| (2) | Cetanol | 2.5 |
| (3) | Isopropyl palmitate | 1.0 |
| (4) | Purified lanolin | 0.5 |
| (5) | Lactic acid | 1.5 |
| (6) | Silicone elastomer powder (Trefil E-500, mfd. by Dow Corning Toray Silicone Co., Ltd., average particle size: 3 µm, true specific gravity: 0.97) | 1.0 |
| (7) | Hydroxyethyl cellulose | 0.5 |
| (8) | High polymerization methylpolysiloxane (6×10 ⁶ cs) | 0.5 |
| (9) | Dimethylpolysiloxane (200 cs) | 0.3 |
| (10) | Preservative, Colorant, Perfume | sufficient quantity |
| (11) | Purified water | balance |

The hair care products obtained in the above Examples 2 to 4 each imparted softness and smoothness to the moist hair and, after drying, made the hair soft, moist and well styled while keeping smooth or oil-free feel and exerting long-lasting conditioning effects thereon.

## Claims

1. A hair care product which comprises an α-carboxylic acid and a water insoluble silicone elastomer rubber powder selected from methyl polysiloxanes, obtainable by polymerising or copolymerising methyl hydrogen polysiloxane, methyl vinyl polysiloxane and/or α-ω-divinyl dimethyl polysiloxane and have rubber elasticity and cross linked structure, and derivatives thereof, selected from amino-modified methyl polysiloxane, phenyl-modified methyl polysiloxane and epoxy-modified methyl polysiloxane, or a composite powder comprising the above-described methyl polysiloxane or the derivatives thereof as the major component together with other water insoluble polymer powders selected from polymethylsilsesquioxanes; amorphous silicone dioxide; polyethylene, polypropylene, polystyrene, polyamide, polyester or styrene/divinylbenzene copolymers, and which is to be washed away after using.

2. The hair care product as claimed in Claim 1, wherein said α-hydroxycarboxylic acid is at least one acid selected from citric acid, lactic acid, malic acid, glycolic acid and tartaric acid.

3. The hair care product as claimed in Claim 1 or 2, wherein said α-hydroxycarboxylic acid is contained in an amount of from 0.01 to 10 % by weight.

4. The hair care product as claimed in any of Claims 1 to 3, wherein said water-insoluble silicone elastomer powder has an average particle size of from 0.01 to 100 µm.

5. The hair care product as claimed in any of Claims 1 to 4, wherein said water-insoluble silicone elastomer powder is contained in an amount of from 0.01 to 10 % by weight.

6. The hair care product as claimed in any of Claims 1 to 5, wherein said water-insoluble silicone elastomer powder has a spherical shape.

7. Use of a hair care product which contains an α-hydroxycarboxylic acid and a water-insoluble silicone elastomer rubber powder according to claim 1 as a shampoo.

8. Use of a hair care product which contains an α-hydroxycarboxylic acid and a water-insoluble silicone elastomer rubber powder according to claim 1 as a rinse or a hair conditioner.

## Patentansprüche

1. Haarpflegeprodukt, umfassend eine α-Hydroxycarbonsäure und ein wasserunlösliches Silicon-Elastomer-Gummipulver, ausgewählt aus Methylpolysiloxanen, erhältlich durch Polymerisation oder Copolymerisation von Methylhydrogenpolysiloxan, Methylvinylpolysiloxan und/oder α-ω-Divinyldimethylpolysiloxan, und mit einer Gummielastizität und einer vernetzten Struktur und Derivate davon, ausgewählt aus Amino-modifiziertem Methylpolysiloxan, Phenyl-modifiziertem Methylpolysiloxan und Epoxy-modifiziertem Methylpolysiloxan, oder ein Verbundpulver, umfassend das oben beschriebene Methylpolysiloxan oder die Derivate davon als Hauptkomponente, zusammen mit anderen wasserunlöslichen Polymerpulvern, ausgewählt aus Polymethylsilsesquioxanen; amorphem Siliciumdioxid; Polyethylen, Polypropylen, Polystyrol, Polyamid, Polyester oder Styrol/Divinylbenzol-Copolymeren, und das nach der Verwendung ausgewaschen wird.

2. Haarpflegeprodukt nach Anspruch 1, worin die α-Hydroxycarbonsäure zumindest eine Säure ist, ausgewählt aus Zitronensäure, Milchsäure, Äpfelsäure, Glykolsäure und Weinsäure.

3. Haarpflegeprodukt nach Anspruch 1 oder 2, worin die α-Hydroxycarbonsäure in einer Menge von 0,01 bis 10 Gew.% enthalten ist.

4. Haarpflegeprodukt nach einem der Ansprüche 1 bis 3, worin das wasserunlösliche Silicon-Elastomer-Pulver eine durchschnittliche Teilchengrösse von 0,01 bis 100 µm hat.

5. Haarpflegeprodukt nach einem der Ansprüche 1 bis 4, worin das wasserunlösliche Silicon-Elastomer-Pulver in einer Menge von 0,01 bis 10 Gew.% enthalten ist.

6. Haarpflegeprodukt nach einem der Ansprüche 1 bis 5, worin das wasserunlösliche Silicon-Elastomer-Pulver eine sphärische Form hat.

7. Verwendung eines Haarpflegeprodukts, umfassend eine α-Hydroxycarbonsäure und ein wasserunlösliches Silicon-Elastomer-Gummipulver nach Anspruch 1, als Shampoo.

8. Verwendung eines Haarpflegeprodukts, umfassend eine α-Hydroxycarbonsäure und ein wasserunlösliches Silicon-Elastomer-Gummipulver nach Anspruch 1, als Spülung oder Haarkonditionierer.

## Revendications

1. Produit de soins capillaires comprenant un acide α-hydroxycarboxylique et une poudre de gomme de silicone élastomérique insoluble dans l'eau choisie parmi les méthyl-polysiloxanes, pouvant être obtenue par polymérisation ou copolymérisation du méthyl-hydrogène-polysiloxane, du méthyl-vinyl-polysiloxane et/ou du α-ω-divinyl-diméthyl-polysiloxane, présentant l'élasticité du caoutchouc et une structure réticulée, ainsi que les dérivés de ceux-ci, choisis parmi le méthyl-polysiloxane amino-modifié, le méthyl-polysiloxane phényl-modifié et le méthyl-polysiloxane époxy-modifié, ou une poudre composite comprenant le méthyl-polysiloxane décrit ci-dessus ou les dérivés de celui-ci comme composant majeur associé à d'autres poudres polymères insolubles dans l'eau choisies parmi les polyméthylsilsesquioxanes, le dioxyde de silicone amorphe, le polyéthylène, le polypropylène, le polystyrène, le polyamide, les copolymères de polyester ou de styrène et de divinylbenzène, produit qui doit être éliminé par rinçage après utilisation.

2. Produit de soins capillaires selon la revendication 1, dans lequel ledit acide α-hydroxycarboxylique est au moins un acide choisi parmi l'acide citrique, l'acide lactique, l'acide malique, l'acide glycolique et l'acide tartrique.

3. Produit de soins capillaires selon les revendications 1 ou 2, dans lequel ledit acide α-hydroxycarboxylique représente de 0,01 à 10 % en poids du produit.

4. Produit de soins capillaires selon l'une quelconque des revendications 1 à 3, dans lequel ladite poudre de silicone élastomérique insoluble dans l'eau présente une grosseur moyenne de particule de 0,01 à 100 µm.

5. Produit de soins capillaires selon l'une quelconque des revendications 1 à 4, dans lequel ladite poudre de silicone élastomérique insoluble dans l'eau représente de 0,01 à 10 % en poids du produit.

6. Produit de soins capillaires selon l'une quelconque des revendications 1 à 5, dans lequel ladite poudre de silicone élastomérique insoluble dans l'eau présente une forme sphérique.

7. Utilisation, en tant que shampooing, d'un produit de soins capillaires contenant un acide α-hydroxycarboxylique et une poudre de gomme de silicone élastomérique insoluble dans l'eau selon la revendication 1.

8. Utilisation, en tant qu'après-shampooing ou baume démêlant, d'un produit de soins capillaires contenant un acide α-hydroxycarboxylique et une poudre de gomme de silicone élastomérique insoluble dans l'eau selon la revendication 1.
